# EUROPÄISCHE PATENTANMELDUNG

(11) **EP 0 600 326 A2**
(43) Veröffentlichungstag der Anmeldung: **08.06.1994**
(21) Anmeldenummer: 93118697.7
(22) Anmeldetag: 20.11.1993
(51) Int. Cl.: C07K 15/00, C07K 3/04, G01N 33/53

(54) **Streptolysin O Peptidantigene und Verfahren zur Bestimmung von Streptolysin-Antikörper**

(30) Priorität: 28.11.1992 DE 4240056
(71) Anmelder: BOEHRINGER MANNHEIM GMBH, D-68305 Mannheim (DE)
(72) Erfinder: Seidel, Christophe, Dr., D-82362 Weilheim (DE); Burns, Geoffrey, Dr., D-80337 München (DE); Engel, Wolf-Dieter, Dr., D-82340 Feldafing (DE)

(57) **Zusammenfassung**

Es werden SLO Peptidantigene beschrieben. Diese Peptidantigene sind zur Bestimmung von SLO-Antikörpern, als Immunogene zur Herstellung von Antikörpern gegen SLO und als Vakzine zur Herstellung von Impfstoffen gegen SLO geeignet.

SEQUENZPROTOKOLL

(1) ALLGEMEINE INFORMATION:

(i) ANMELDER:
(A) NAME: Boehringer Mannheim GmbH
(B) STRASSE: Sandhofer Strasse 116
(C) ORT: Mannheim
(E) LAND: DE
(F) POSTLEITZAHL: 68298
(G) TELEPHON: 0621-759-4337
(H) TELEFAX: 0621-759-4457

(ii) ANMELDETITEL: Streptolysin O Peptidantigene und
Verfahren zur Bestimmung von
Streptolysin-Antikoerper

(iii) ANZAHL DER SEQUENZEN: 5

(iv) COMPUTER-LESBARE FORM:
(A) DATENTRÄGER: Floppy disk
(B) COMPUTER: IBM PC compatible
(C) BETRIEBSSYSTEM: PC-DOS/MS-DOS
(D) SOFTWARE: PatentIn Release #1.0,
Version #1.25 (EPA)

(2) INFORMATION ZU SEQ ID NO: 1:

(i) SEQUENZ CHARAKTERISTIKA:
(A) LÄNGE: 11 Aminosäuren
(B) ART: Aminosäure
(C) STRANGFORM: Einzel
(D) TOPOLOGIE: linear

(ii) ART DES MOLEKÜLS: Peptid

(xi) SEQUENZBESCHREIBUNG: SEQ ID NO: 1:

Pro Lys Pro Glu Ser Ser Glu Leu Thr Thr Glu
1               5                   10

(2) INFORMATION ZU SEQ ID NO: 2:

(i) SEQUENZ CHARAKTERISTIKA:
(A) LÄNGE: 13 Aminosäuren
(B) ART: Aminosäure
(C) STRANGFORM: Einzel
(D) TOPOLOGIE: linear

(ii) ART DES MOLEKÜLS: Peptid

(xi) SEQUENZBESCHREIBUNG: SEQ ID NO: 2:

Gln Lys Thr Asp Asp Met Leu Asn Ser Asn Asp Met Ile
1               5                   10

(2) INFORMATION ZU SEQ ID NO: 3:

(i) SEQUENZ CHARAKTERISTIKA:
(A) LÄNGE: 11 Aminosäuren
(B) ART: Aminosäure
(C) STRANGFORM: Einzel
(D) TOPOLOGIE: linear

(ii) ART DES MOLEKÜLS: Peptid

(xi) SEQUENZBESCHREIBUNG: SEQ ID NO: 3:

Ile Lys Leu Ala Pro Lys Glu Met Pro Leu Glu
1               5                   10

(2) INFORMATION ZU SEQ ID NO: 4:

(i) SEQUENZ CHARAKTERISTIKA:
(A) LÄNGE: 13 Aminosäuren
(B) ART: Aminosäure
(C) STRANGFORM: Einzel
(D) TOPOLOGIE: linear

(ii) ART DES MOLEKÜLS: Peptid

(xi) SEQUENZBESCHREIBUNG: SEQ ID NO: 4:

Glu Met Pro Leu Glu Ser Ala Glu Lys Glu Glu Lys Lys
1               5                   10

(2) INFORMATION ZU SEQ ID NO: 5:

(i) SEQUENZ CHARAKTERISTIKA:
(A) LÄNGE: 11 Aminosäuren
(B) ART: Aminosäure
(C) STRANGFORM: Einzel
(D) TOPOLOGIE: linear

(ii) ART DES MOLEKÜLS: Peptid

(xi) SEQUENZBESCHREIBUNG: SEQ ID NO: 5:

Tyr Asp Asp Lys Gly Lys Glu Val Ile Thr Lys
1               5                   10

## Beschreibung

Die Erfindung betrifft Streptolysin Peptidantigene, ein Verfahren zur Herstellung dieser Peptidantigene, sowie ein Verfahren zur Bestimmung von Streptolysin-Antikörper unter Verwendung der Peptidantigene.

Streptolysin O (SLO) ist ein Protein aus Streptokokken, welches eine Zellyse durch Bildung von Tunnelstrukturen in der Zellmembran bewirkt. Streptolysin O ist ein Thiolaktivierbares Toxin und stellt einen bedeutenden Parameter zum Nachweis von bakterieller Virulenz dar. Nach einer Infektion durch Streptokokken werden im Blut Antikörper gegen Streptolysin beobachtet. Der Nachweis einer Streptokokkeninfektion wäre demnach durch einen immunologischen Test möglich, in dem Peptide verwendet werden, die mit hoher Affinität an solche Antikörper binden und mit denen zuverlässig diese Antikörper detektiert werden können. Die DNA- und Aminosäuresequenz von Streptolysin O ist aus M. A. Kehoe, Infection and Immunity 55 (1987), 3228 - 3232 bekannt. Streptolysin O besteht aus 571 Aminosäuren und hat ein Molekulargewicht von 63.645 D. Streptolysin O besitzt am N-Terminus ein 33 Aminosäuren langes Signalpeptid, welches bei der Sekretion von SLO abgespalten wird, wobei ein reifes Peptid mit 538 Aminosäuren Länge und einem Molekulargewicht von 60.151 D entsteht. Außerdem wird aufgrund von in vitro Experimenten vermutet, daß natives SLO proteolytisch gespalten wird, wobei ein Fragment mit einem Molekulargewicht von 7.000 D zusätzlich abgespalten wird.

Die Aminosäuresequenz des bei der Sekretion abgespaltenen N-terminalen Teils von SLO wurde bisher nicht durch Sequenzanalyse bestimmt, sondern auf Grund der Nukleotidsequenz der prepro-Form ermittelt. Der Grund hierfür liegt darin, daß dieses N-terminale Fragment vermutlich in äußerst geringen Mengen in Körperflüssigkeiten, wie beispielsweise im Blut, vorliegt.

Die Bestimmung von Antikörpern gegen Streptolysin O (Gruppe A) wird in der Diagnose von Streptokokkeninfektionen, die beispielsweise Gelenkrheumatismus und Glomerulonephritis verursachen, verwendet. Dieser sogenannte ASLO-Test ist besonders nützlich in der Differenzialdiagnose von Gelenkrheumatismus und Rheumatismus.

Der Nachweis von Streptolysin O erfolgt derzeit üblicherweise durch Hämolysetests (beispielsweise mit Schaferythrozyten) oder durch Latexagglutinationstests, (J. Clin. Microbiol. 26 (1988) 1406-1408), welche sehr zeitaufwendig und nicht automatisierbar sind.

Aufgabe der vorliegenden Erfindung ist es deshalb, Antigene zur Verfügung zu stellen, die spezifisch für Antistreptolysin O Antikörper sind und für einfache immunologische Tests auf Antistreptolysin-Antikörper geeignet sind.

Die Aufgabe wird gelöst durch Streptolysin O Peptidantigene, die im CD-Spektrum eine negative Bande bei 190-200 nm zeigen und mindestens eine der Aminosäuresequenzen
SEQ ID NO:1 PKPESSELTTE
SEQ ID NO:2 QKTDDMLNSNDMI
SEQ ID NO:3 IKLAPKEMPLE
SEQ ID NO:4 EMPLESAEKEEKK
SEQ ID NO:5 YDDKGKEVITK
oder Teilsequenzen davon mit mindestens 4 Aminosäuren Länge enthalten.

Wesentlich für die Eignung der erfindungsgemäßen Peptidantigene ist, daß sie nicht in einer definierten gefalteten Struktur, sondern linear vorliegen. Eine solche lineare Struktur wird auch als "random coil" Struktur bezeichnet. Ob für ein Oligopeptid eine solche Struktur vorliegt, läßt sich durch CD-Spektroskopie überprüfen. Ein Peptid mit "random coil"-Struktur besitzt zwischen 190 und 200 nm im CD-Spektrum eine negative Bande (vgl. Ann. Rev. Biophys. Chem. 17 (1988) 145-166).

Es hat sich gezeigt, daß die Peptide SEQ ID NO: 2 oder 5 oder die Peptidkombination SEQ ID NO: 1, 3 und 4 einen spezifischen Nachweis von Anti SLO-Antikörpern ermöglichen. Durch Kombination mit den anderen erfindungsgemäßen Peptidantigenen kann die Sensitivität weiter erhöht werden.

Bevorzugte Kombinationen (Mischungen) sind
SEQ ID NO: 2, 5
SEQ ID NO: 1, 2, 3, 4
SEQ ID NO: 1, 2, 3, 4, 5
SEQ ID NO: 1, 3, 4, 5
Bevorzugt werden die oben unterstrichenen Teilsequenzen verwendet. Besonders bevorzugt werden Teilsequenzen, deren Länge maximal 7 Aminosäuren, in Mischungen 4 - 7 Aminosäuren betragen, verwendet. Die Bestimmung von Streptolysin-Antikörpern erfolgt zweckmäßig dadurch, daß durch immunologische Tests Antikörper gegen Streptolysin in Körperflüssigkeiten nachgewiesen werden. Für derartige immunologische Tests werden deshalb Bindepartner für Anti-Streptolysin-Antikörper benötigt.

Die Durchführung eines Anti-SLO-Antikörper-Nachweis erfolgt nach den dem Fachmann geläufigen Methoden. Ein weiterer Gegenstand der Erfindung ist deshalb ein Verfahren zur Bestimmung von SLO-Antikörpern, welches dadurch gekennzeichnet ist, daß die Probe mit den Peptidantigenen oder Teilsequenzen davon mit mindestens 4, vorzugsweise 4 - 7 Aminosäuren, der SEQ ID NO: 2 und/oder 5 oder mit der Peptidkombination SEQ ID NO: 1, 3 und 4 inkubiert und unter Bedingungen, die die Bildung eines Antikörper-Antigenkomplexes ermöglichen, die Menge der an das Peptidantigen gebundenen Anti-SLO-Antikörper bestimmt wird. Bevorzugt werden die unterstrichenen Teilsequenzen verwendet.

Bevorzugt wird eine Kombination von SEQ ID NO: 2 und/oder 5 bzw. Teilsequenzen davon mit mindestens einem Peptidantigen der SEQ ID NO: 1, 3 und 4 eingesetzt.

Ebenfalls bevorzugt ist es, die Sequenzen SEQ ID NO: 1 - 5 oder Teilsequenzen davon mit beliebigen Aminosäuren zu einem synthetischen Oligopeptid zu kombinieren und diese erfindungsgemäß zu verwenden. Dabei muß lediglich darauf geachtet werden, daß das Antigen eine "random coil"-Struktur behält.

Die Kombination der Peptidantigene kann beispielsweise dadurch erfolgen, daß mehrere einzelne Peptidantigene verwendet werden oder daß die Peptidantigene kovalent, zweckmäßig über eine Aminosäurenbrücke, die sich auch von natürlicherweise in SLO-Proteinen vorkommenden Aminosäuren-Sequenzfolgen unterscheiden kann oder einen Peptid-Linker darstellen kann, aneinander gebunden werden.

Die Peptidantigene ID NO: 2 oder 5 werden allein in Konzentrationen von vorzugsweise 0,2 - 1 µg/ml eingesetzt.

In Kombinationen werden die Peptidantigene vorzugsweise in einer Menge von 0,01 - 1 µg/ml, besonders bevorzugt in folgenden Mengen verwendet:

| Peptid-antigen | Menge in Kombination [µg/ml] | |
|---|---|---|
| | Bereich | besonders bevorzugter Bereich |
| 1 | 0.02 - 1.50 | 0.20 - 0.50 |
| 2 | 0.01 - 0.50 | 0.05 - 0.20 |
| 3 | 0.05 - 2.50 | 0.50 - 0.80 |
| 4 | 0.02 - 1.50 | 0.20 - 0.50 |
| 5 | 0.01 - 0.50 | 0.05 - 0.20 |

Zum Nachweis werden heterogene und homogene Immunoassays verwendet. Die heterogenen Tests erlauben Waschschritte, die den Meßsignal-Hintergrund erheblich reduzieren wodurch die Empfindlichkeit gesteigert werden kann.

Beispielsweise kann die Bestimmung durch einen Radio-Immunoassay, Enzym-Immunoassay oder durch Immunfluoreszenz erfolgen. Üblicherweise wird hierzu das Peptidantigen immobilisiert. Die Probe, welche auf Anti-SLO-Antikörper untersucht werden soll, wird zugegeben und die an das Antigen gebundenen Antikörper über einen markierten Anti-Human-Immunglobulin-Antikörper bestimmt. Die Immobilisierung des erfindungsgemäßen Peptidantigens kann adsorptiv, kovalent oder über ein biologisches Bindungspaar wie Biotin/Streptavidin, Antikörper/Antigen, oder Zucker/Lektin erfolgen. Dabei wird das Peptidantigen an einen dieser Partner kovalent gebunden.

Die erfindungsgemäßen Peptidantigene können für Immunoassays vorzugsweise nach dem Fachmann geläufigen Methoden, beispielsweise an Kugeln (beads), Kunststoffröhrchen oder Mikrotiterplatten (bevorzugt aus Polystyrol oder Copolymere von Polystyrol), immobilisiert werden. Dies erfolgt vorzugsweise dadurch, daß das Peptidantigen unspezifisch an der Oberfläche adsorbiert oder kovalent an funktionalisierte oder aktivierte Oberflächen gebunden wird. Die unspezifische Adsorption kann dadurch verbessert werden, daß man das Peptidantigen mit einem Protein zu einem Konjugat verknüpft und dieses Konjugat zur Adsorption verwendet (vgl. z.B. EP-A 0 269 092). Die Bindung kann auch über einen immobilisierten Antikörper erfolgen. Dabei sollte darauf geachtet werden, daß durch diese Bindung des Antikörpers bzw. Proteins an das Peptidantigen das Epitop nicht blockiert wird.

Die Konjugation des Peptidantigens an den Bindungspartner erfolgt vorzugsweise über einen Spacer. Dieser Spacer enthält zweckmäßig 10 - 50, vorzugsweise 10 - 30 Atome und ist ebenfalls bevorzugt ein im wesentlichen lineares Molekül. Beispiele hierfür sind Spacer aus Alkyl-, Polyether- oder Polyamidketten. In einer besonders bevorzugten Ausführungsform ist ein Peptidantigen einer Länge von 4 - 9 Aminosäuren über einen linearen Spacer von 10 - 30 Atomen an den Träger gebunden. Falls ein Spacer aus Aminosäuren verwendet werden soll, besteht dieser zweckmäßig aus Aminosäuren, die nicht der Sequenzfolge in unmittelbarer Umgebung des Peptidantigens im SLO-Gen entsprechen.

In einer bevorzugten Ausführungsform wird das erfindungsgemäße Peptidantigen kovalent an Biotin gebunden, wobei die Immobilisierung über eine Avidin/Streptavidin-Festphase erfolgt.

Ebenso geeignet sind Bestimmungsverfahren, bei denen die Detektion nicht über einen markierten Antikörper, sondern über ein markiertes, weiteres Peptidantigen der SEQ ID NO 1 - 5 oder Teilsequenzen davon erfolgt.

Eine weitere besonders vorteilhafte Ausführungsform des Assays unter Verwendung dieser Peptide ist ein Immunoassay, bei dem beschichtete Partikel eingesetzt werden (LPIA). Bei diesem System werden Teilchen, die mit einzelnen Peptiden oder einer Mischung der Peptide überzogen sind, mit der Probe vermischt. Die Teilchen werden in Gegenwart von gegen SLO gerichteten Antikörpern agglutiniert. Der Agglutinationsgrad kann qualitativ mit dem bloßen Auge gemessen oder mit Hilfe eines Photometers, Nephelometers oder eines anderen geeigneten Meßinstruments quantifiziert werden.

Es können verschiedene Partikel verwendet werden. Besonders bevorzugt ist die Verwendung von Latexteilchen. Jedoch können eine Reihe anderer Teilchen eingesetzt werden, darunter beispielsweise Metallsole (z.B. Goldsole), Liposome oder Polystreptavidin.

Die Beschichtung von Teilchen (vorzugsweise Latexpartikel) mit Antikörpern und Antigenen kann nach den dem Fachmann geläufigen Methoden erfolgen. In der Regel erfolgt die Beschichtung entweder durch Adsorption oder kovalente Bindung. Jedoch ist auch die Verwendung von biologischen Bindungspaaren möglich. Besonders vorteilhaft ist die Verwendung biotinylierter Peptide, die auf Avidin/-Streptavidin-Latexteilchen aufgetragen werden können.

Die Beschichtung der Latices mit Antigenen erfolgt vorteilhaft über einen Spacer. Geeignete Spacer sind weiter oben beschrieben.

Eine weitere vorteilhafte Ausführungsform des Immunoassays zum Nachweis von Anti-SLO-Antikörpern unter Verwendung dieser Peptide ist das FPIA, EMIT oder CEDIA-Prinzip.

Beim Fluoreszenz-Polarisations-Immunoassay (FPIA) wird das erfindungsgemäße Peptidantigen mit einer fluoreszierenden Substanz markiert. Diese Moleküle absorbieren Lichtenergie und geben sie in einem Zeitraum von etwa 10⁻⁸ sec als Licht längerer Wellenlänge wieder ab. Wird der Fluorophor durch polarisiertes Licht angeregt, so hängt der Polarisationsgrad des emittierten Lichtes von der Rotationsgeschwindigkeit des Tracers (Peptidantigen-Fluorophor-Konjugat) ab. Die Bindung des Tracers an einen Antikörper behindert die Rotation des Fluorophores. Der freie Tracer rotiert schneller und depolarisiert das anregende Licht mehr als der größere, trägere Antikörper-Tracer-Komplex. Je mehr Anti-SLO-Antikörper in der Probe vorhanden sind, desto mehr Antikörper-Tracer-Komplexe entstehen und desto mehr Fluoreszenzpolarisation ist meßbar. (W. Dandliker et al., Journal of Exp. Med. 122 (1965), 1029).

Bei der Enzyme Multiplied Immunoassay Technique (EMIT) wird das Peptidantigen so kovalent mit dem Markerenzym gekoppelt, daß die Enzymativität erhalten bleibt. Nach Bindung eines Anti-SLO-Antikörpers an den Peptidantigen-Anteil wird die Substratbindung an das Enzym sterisch jedoch behindert, so daß keine enzymatische Umsetzung des Substrats erfolgen kann. Die enzymatische Aktivität ist somit umgekehrt proportional zur Konzentration des zu analysierenden Anti-SLO-Antikörpers in der Probelösung (Gunzer et al., Kontakte III, 1980, 3 - 11 und K. Rubenstein, Biochemical and Biophysical Research Communications 47 (1972), 846 - 851).

Beim CEDIA-Prinzip (Henderson et al., Clinical Chemistry 32 (1986), 1637 - 1641) werden zum Nachweis bestimmte Enzyme wie zum Beispiel die β-Galactosidase verwendet, die in zwei jeweils enzymatisch inaktiven Bestandteilen, nämlich einem großen Polypeptid (Enzymakzeptor) und einem kleinen Polypeptid (Enzymdonor) vorliegen, wobei diese Bestandteile spontan zu einem enzymatisch aktiven Protein assoziieren. An den Enzymdonor wird das Peptidantigen derart gebunden, daß die Assoziation des Enzymdonors mit dem Enzymakzeptor zum aktiven Enzym durch diese Bindung nicht verhindert wird. Diese Assoziation wird aber dann gehemmt, wenn an den Peptidantigen-Enzymdonor-Komplex ein Antikörper gegen das Peptidantigen bindet. In einer Reagenzlösung, in der Enzymakzeptor und Peptidantigen-Enzymdonor-Komplex vorliegen, kann daher aktives Enzym gebildet werden und es wird enzymatische Aktivität gemessen. Nach Zugabe der Probelösung bindet der Anti-SLO-Antikörper aus dieser Probelösung an den Peptidantigen-Enzymdonor-Komplex und verhindert so die Ausbildung des aktiven Enzyms. Das Meßsignal ist somit umgekehrt proportional der Menge des vorhandenen Antikörpers.

Die Herstellung der erfindungsgemäßen Peptidantigene ist nach den dem Fachmann geläufigen Methoden zur Peptidsynthese möglich. Ein weiterer Gegenstand der Erfindung ist deshalb ein Verfahren zur Herstellung des erfindungsgemäßen Peptidantigens, welches darin besteht, daß die das C-terminale Ende bildende Aminosäure an einen Träger gebunden wird, vom C-terminalen Ende das erfindungsgemäße Peptidantigen schrittweise aufgebaut und dieses anschließend vom Träger abgespalten wird.

Im einzelnen wird dazu eine Aminosäure beispielsweise über ihre Carboxygruppe an ein unlösliches, leicht filtrierbares Polymer geknüpft, und dann vom C-terminalen Ende her die Peptidkette schrittweise aufgebaut. Zu diesem Zweck wird eine N-geschützte Aminosäure mit einer reaktiven Gruppierung des Kunstharzes zur Reaktion gebracht. Von der am Trägerpartikel kovalent verankerten Aminosäure wird die Nα-Schutzgruppe entfernt und das resultierende Aminoacylpolymer mit der nächsten N-geschützten Aminosäure umgesetzt. Von dem am Trägerharz kovalent gebundenen Dipeptid wird die Nα-Schutzgruppe entfernt und das resultierende Aminoacylpolymer mit der nächsten N-geschützten Aminosäure umgesetzt. Alle überschüssigen Reagentien und Beiprodukte werden durch einfaches Filtrieren entfernt. Ist die gewünschte Peptidsequenz auf diese Weise hergestellt, wird die kovalente Bindung zwischen der C-terminalen Aminosäure und der Ankergruppierung des polymeren Trägers gespalten. Der unlösliche Träger wird durch einfache Filtration von dem nun in Lösung befindlichen Peptid entfernt. Das Peptid wird mittels chromatographischer Methoden gereinigt.

Beispielsweise können die erfindungsgemäßen Peptidantigene nach Merrifield, JACS 85(1964)2146 hergestellt werden. Eine gegebenenfalls erforderliche Biotinylierung kann beispielsweise nach PNAS USA 80 (1983) 4045 erfolgen. Ein bevorzugtes Biotinylierungsmittel hierfür ist Biotinylaminocapronsäure-N-hydroxysuccinimidester.

Ein bevorzugtes Verfahren zur Herstellung von biotinylierten Peptidantigenen ist die Einführung des Biotinrestes am N-Terminus während einer Festphasensynthese des Peptidantigens.

Dieses Verfahren wird bevorzugt dann verwendet, wenn das Peptidantigen mehrere ε-Lysin-Aminogruppen enthält, die nicht biotinyliert werden sollen. Dies ist beispielsweise dann der Fall, wenn man N-α-Fmoc-N-ε-biotinyl-aminocaproyl)lysin, N-α-Fmoc-N-ε-Biotinyllysin oder bei Biotinylierung der N-terminalen Aminosäuren Biotin, Biotinylaminocapronsäure oder Dimethoxytritylbiotin mit einem Aktivierungsreagenz wie zum Beispiel Dicyclohexylcarbodiimid oder als Aktivester einsetzt.

In einer weiteren bevorzugten Ausführungsform wird ein Nachweisantikörper der beispielsweise gegen den Fc-Teil von humanem IgG gerichtet ist, immobilisiert. Vorzugsweise wird hierzu ein monoklonaler Antikörper verwendet. Das Peptidantigen befindet sich dann in Lösung. Der nachzuweisende Antikörper (Analyt) und auch alle anderen Antikörper der Probenflüssigkeit werden vom Wandantikörper gebunden. Der gebundene Antikörper kann dann den Analyten binden, der mit einem geeigneten Nachweissystem, z.B. kompetitiv mit einem Peptidantigen-Enzymkonjugat nachgewiesen werden kann.

Mit den erfindungsgemäßen Peptidantigenen ist es auch möglich, durch die dem Fachmann geläufigen Verfahren der Immunisierung Antikörper zu erhalten, mit denen das infektiöse Partikel selbst in einem immunologischen Test nachgewiesen werden kann.

Ein weiterer Gegenstand der Erfindung ist deshalb ein Verfahren zur Herstellung von Antikörpern, welches dadurch gekennzeichnet ist, daß ein Säugetier mit einem erfindungsgemäßen Peptid, welches gegebenenfalls an einen Träger gebunden ist, immunisiert wird und die Antikörper nach bekannten Verfahren z.B. aus dem Serum oder der Milz gewonnen werden.

In einer bevorzugten Ausführungsform werden B-Lymphozyten der immunisierten Tiere in Gegenwart von transformierenden Agenzien mit einer geeigneten Zellinie fusioniert, die Zellinie, welche die gewünschten Antikörper produziert, kloniert und kultiviert und aus den Zellen oder dem Kulturüberstand die monoklonalen Antikörper gewonnen.

Die so hergestellten Anti-SLO-Antikörper können beispielsweise als Teststandards in den Immunoassays zum Nachweis von Anti-SLO-Antikörpern eingesetzt werden. Weiterhin können die so hergestellten Anti-SLO-Antikörper in einem immunologischen Test zum Nachweis des SLO-Antigens selbst verwendet werden.

Ein weiterer Gegenstand der Erfindung ist deshalb ein Verfahren zur Bestimmung von SLO, welches dadurch gekennzeichnet ist, daß die Probe mit einem erfindungsgemäßen Antikörper unter Bedingungen, die eine Antigen-Antikörperkomplexbildung erlauben, inkubiert, und die Menge des gebildeten Antikörper-Antigenkomplexes bestimmt wird.

Der Nachweis des gebildeten Antikörper-Antigen-Komplexes kann mit allen, dem Fachmann geläufigen Methoden wie Enzym-Immunoassay, Radio-Immunoassay, Immunfluoreszenz wie FPIA, homogene Immunoassays wie CEDIA oder EMIT oder turbidimetrische und nephelometrische Bestimmungsmethoden geführt werden. Im Gegensatz zu den FPIA, CEDIA und EMIT-Verfahren zur Bestimmung von Anti-SLO-Antikörpern, wie vorne beschrieben, werden zur Bestimmung von SLO kompetitive Testvarianten dieser Verfahren, wie sie dem Fachmann bekannt sind, benutzt. Besonders bevorzugt sind kompetitive Tests, bei denen neben den erfindungsgemäßen Antikörpern die erfindungsgemäßen Peptidantigene als Haptene oder Polyhaptene oder Bestandteile des Enzymdonors (CEDIA), Tracers (FPIA) oder Markerenzyms (EMIT) eingesetzt werden. Besonders bevorzugt sind kompetitive turbidimetrische Bestimmungsmethoden. Hierzu werden einzelne oder mehrere erfindungsgemäße Peptidantigene an einen Träger gekoppelt und der erhaltene Komplex als Hapten im Agglutinationstest eingesetzt. Durch den zu bestimmenden Analyten wird dies Trübung dann in einem zur Menge des Analyten proportionalen Ausmaß reduziert. Vorzugsweise werden 30 bis 40 Peptidantigene je Trägermolekül gekoppelt. Die Bindung des erfindungsgemäßen Peptidantigens an den Träger kann dabei direkt über kovalente Bindungen erfolgen oder aber indirekt, zum Beispiel durch Biotinylierung des Peptidantigens und eine Streptavidinbeschichtung des Trägermaterials. Als Trägermolekül werden dabei vorzugsweise Proteine wie Immunglobuline, Albumin, β-Galaktosidase, Polymere wie Aminodextrane oder Polylysine oder Partikel wie Latex oder Gold, jeweils allein oder in Kombination miteinander, verwendet. Die Kopplung an das Trägermolekül erfolgt in bekannter Weise, zum Beispiel mit Reagenzien wie Glutardialdehyd, Ethyldimethylaminopropylcarbodiimid, Maleinimidohexansäure-N-hydroxysuccinimidester oder anderen bekannten homo- oder heterobifunktionellen Linkern.

In den immunologischen Tests zur quantitativen Bestimmung von SLO können die erfindungsgemäßen Peptidantigene als Standard verwendet werden. Ein weiterer Gegenstand der Erfindung ist daher die Verwendung der erfindungsgemäßen Peptidantigene als Standard in einem immunologischen Test zur Bestimmung von SLO. In bestimmten Fällen, wie zum Beispiel bei Agglutinationstests, kann es dabei von Vorteil sein, mehrere erfindungsgemäße Peptide gleicher oder unterschiedlicher Sequenz an ein Trägermolekül zu binden.

Ein weiterer Gegenstand der Erfindung ist ein Verfahren zur Herstellung von Impfstoffen unter Verwendung der erfindungsgemäßen Peptidantigene sowie ein Vakzin zur Behandlung von Streptokokken-Infektionen, enthaltend als Immunogen ein gegebenenfalls trägergebundenes Peptidantigen der Sequenzen SEQ ID NO: 2 oder 5 oder die Kombination SEQ ID NO: 1,3,4 oder Teilsequenzen davon in einer pharmakologisch effektiven Dosis und in einer pharmazeutisch akzeptablen Formulierung.

Die Herstellung dieser Impfstoffe kann nach den bekannten Methoden durchgeführt werden. Bevorzugt werden jedoch die Peptidantigene zunächst lyophilisiert und anschließend, ggf. unter Zusatz von Hilfsstoffen, suspendiert.

Die Impfung mit dem erfindungsgemäßen Vakzin oder Vakzinkombinationen kann durch die dem Fachmann geläufigen Methoden erfolgen, beispielsweise intradermal, intramuskulär, intraperitoneal, intravenös, subkutan und intranasal.

Zur intramuskulären oder subkutanen Gabe kann das Vakzin beispielsweise in physiologischer Kochsalzlösung suspendiert sein. Zur intranasalen oder intraoccularen Applikation kann das Vakzin, beispielsweise in Form eines Sprays oder einer wäßrigen Lösung angewendet werden. Für lokale, beispielsweise orale Gabe ist es häufig erforderlich, die Immunogene zeitweise gegen Inaktivierung zu schützen, beispielsweise gegen proteolytische Enzyme in der Mundhöhle oder im Magen. Ein derartiger vorübergehender Schutz kann beispielsweise durch Verkapselung der Immunogene erfolgen. Diese Verkapselung kann beispielsweise durch Überziehen mit einem Schutzmittel (Mikroverkapselung) oder bei Einbettung einer Vielzahl von erfindungsgemäßen Immunogenen in einen schützenden Träger (Makroverkapselung) erfolgen.

Das Verkapselungsmaterial kann semipermiabel sein oder beim Einbringen in den menschlichen oder tierischen Körper semipermeabel werden. Üblicherweise wird für die Verkapselung eine biologisch abbaubare Substanz als Träger verwendet.

Die nachfolgenden Beispiele und Sequenzprotokolle erläutern die Erfindung weiter.

### Beispiel 1

### Synthese von H-PESSELTTE-OH(NS5/1)

Das Peptid wurde mittels Fmoc(Fluorenyloxycarbonyl)-Festphasensynthese hergestellt. Die Reaktionen wurden an einem Labortec (Schweiz) SP 640 Peptidsynthesizer durchgeführt. Die Kupplungsreaktionen wurden bezüglich Fmoc-Aminosäure-Derivat mit 1.2 Äquivalenten Dicyclohexylcarbodiimid und 1.1 Äquivalenten N-Hydroxybenzotriazol während 90 Minuten durchgeführt. Als Reaktionsmedium kam Dimethylformamid zum Einsatz. Die Fmoc-Gruppe wurde mittels 20 % Piperiden in DMF in 10 und 20 Minuten abgespalten. 4.0 Äquivalente von den folgenden Aminosäure-Derivaten kamen zum Einsatz: Pro, Ser(mit tert.-Butyl-Schutzgruppe), Thr(mit tert.-Butyl-Schutzgruppe), Glu(mit tert. Butylester-Schutzgruppe) und Leu. Der Kupplungserfolg wurde mittels Kaiser-Test (Anal. Biochemistry 34(1970)595) geprüft, die Harzbeladung wurde mittels UV-Absorption der freigesetzten Fulven-Gruppe nach jeder Piperidin-Spaltung ermittelt. Das Peptid wurde an 5 g Wang-Harz (Polystyrol/1 % Divinylbenzol) mit einer Ladung von 0.50 mMol/g synthetisiert (JACS 95(1973)1328). Nach der Synthese betrug der Beladungsgrad noch 0.43 mMol/g.

Die Freisetzung des Peptids erfolgte mit 200 ml Trifluoressigsäure, 20 ml Ethandithiol, 20 ml m-Kresol und 10 ml Wasser in 30 Minuten bei Raumtemperatur. Die Abspaltlösung wurde mehrmals mit Toluol eingeengt, dann das Peptid mit Diethylether gefällt.

Zur Entfernung der Scavenger und anderer kleiner Moleküle wurde das Rohmaterial über eine Sephadex G10-Säule gereinigt. Es wurden nach Lyophilisieren 2.4 g Material einer Reinheit von 54 % (RP-HPLC) erhalten. Um das Material auf die Endreinheit von >95 % zu bringen, wurden 400 mg Peptid über eine präparative RP-HPLC-Säule (40mmx250mm) gefüllt mit C18-Material (5 Mikrometer, 300 Angström) und einem Wasser/Trifluoressigsäure-, Acetonitril/Trifluoressigsäure-Gradienten gereinigt. Es fielen nach Lyophilisation 87 mg 97.2 %iges (HPLC) weißes Material an. Die Identität des Materials wurde mittels FAB-MS geprüft.

### Beispiel 2

Zur Biotinylierung des Peptidantigens aus Beispiel 1 wurde ein Moläquivalent möglichst konzentriert (Löslichkeit ist von der Aminosäuresequenz abhängig) in Argon-gesättigtem Kaliumphosphatpuffer (0,1 mol/l, pH 8,0) gelöst und mit 3 Äquivalenten D-Biotinyl-ε-aminocapronsäure-N-hydroxysuccinimidester gelöst in Argon-gesättigtem Dimethylformamid (Lösung von 1 µmol Reagenz in 5 µl DMF) versetzt.

Man rührte das Reaktionsgemisch 2 Stunden bei Raumtemperatur unter Argon unter ständiger Kontrolle mittels analytischer RP-HPLC. Wenn < 5% Edukt vorhanden waren, wurde der Reaktionsansatz direkt auf eine präparative RP-HPLC-Säule gegeben und das Produktmaterial mittels einem 0.1% Trifluoressigsäure/Wasser- zu 0.1% Trifluoressigsäure/ Acetonitril-Gradienten (Steigung: 0% auf 100% in 90 Minuten) gereinigt. Das Produktmaterial wurde durch Einengen und Lyophilisation der Produktfraktionen erhalten. Die Ausbeuten lagen zwischen 40 % und 90%. Als Analytik dienten für die Reinheit HPLC, HPCE und TLC, für die Identität FAB-MS (Molpeak) und TLC mit spezifischen Anfärbereagentien (p-Dimethylaminozimtaldehyd auf Biotin) und Gehaltsbestimmung über Mikroanalyse (Nitrogen).

### Beispiel 3

SLO-Antikörper werden in einem 2-Schritt-Sandwich-Immunoassay bestimmt. Zum Nachweis werden die Reagentien mit folgender Zusammensetzung verwendet:

### Reagenz 1:

Kombination von biotinylierten Peptidantigenen oder einzelne biotinylierte Antigene.
40 mmol/l Phosphatpuffer pH 7.0
0.9 Gew.-% NaCl
10 Vol.-% Rinderserumalbumin

### Reagenz 2:

20 mU/ml eines Konjugats aus polyklonalem Antikörper gegen humanes Immunglobulin (Schaf) und Peroxidase
40 mmol/l Phosphatpuffer pH 7.0
0.05 Gew.-% Tween 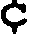 20
0.2% Rinderserumalbumin
0.2% Rinder-IgG
In einem mit Streptavidin beschichteten Polystyrolröhrchen (hergestellt nach Beispiel 1 von EP-A 0 344 578) werden 1 ml Reagenz 1 und 10 µl Probe eine Stunde bei Raumtemperatur inkubiert. Anschließend wird dreimal mit Leitungswasser gewaschen und mit 1 ml Reagenz 2 für eine Stunde bei Raumtemperatur inkubiert. Anschließend wird dreimal mit Leitungswasser gewaschen. Zur Nachweisreaktion werden 1 ml ABTS 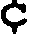 (2,2'-Azino-di-[3-ethyl-benzthiazolinsulfonat(6)]-Diammoniumsalz, 1,9 mmol/l, in 100 mmol/l Phosphatcitratpuffer pH 4.4 mit 3.2 mmol/l Natrium-perborat) zugegeben. Nach 60 min wird die Extinktion bei 420 nm photometrisch gemessen. Die Ergebnisse zeigt Tabelle I.

Erläuterungen zu den Tabellen:
- -/+:: negativ/positiv (Der Cutoff für ein positives Signal im ELISA ist definiert als die mittlere Extinktion bei 420 nm plus 3 Standardabweichungen eines Kollektivs von 10 negativen Kontrollseren. Die Proben wurden bei einer Probenverdünnung von 1:100 vermessen).

Als Antigenkonzentrationen wurden verwendet:
a) als einzelnes Antigen im Test

| Antigen | Menge [µg/ml] |
|---|---|
| 1 | 0.35 |
| 2 | 0.20 |
| 3 | 0.60 |
| 4 | 0.35 |
| 5 | 0.20 |

b) in den Kombinationen

| Antigen | Menge [µg/ml] in Kombination | | | | |
|---|---|---|---|---|---|
| | 1 | 2 | 3 | 4 | 5 |
| 1+2+3+4+5 | 0.20 | 0.10 | 0.40 | 0.20 | 0.10 |
| 2+5 | - | 0.20 | - | - | 0.20 |
| 1+2+4+5 | 0.20 | 0.10 | - | 0.20 | 0.10 |
| 1+3+4 | 0.25 | - | 0.50 | 0.25 | - |

**Tabelle I**

| Serum | SEQ ID NO: | | | | |
|---|---|---|---|---|---|
| | 1 | 2 | 3 | 4 | 5 |
| 1 | + | + | + | + | + |
| 2 | + | + | + | + | + |
| 3 | + | + | - | + | + |
| 4 | + | + | - | + | + |
| 5 | + | + | - | + | + |
| 6 | - | + | + | - | + |
| 7 | + | + | + | +? | + |
| 8 | - | + | - | + | + |
| 9 | + | + | + | + | + |
| 10 | - | + | - | + | (+) |
| 11 | + | + | - | + | (+) |
| 12 | + | + | - | + | + |
| 13 | - | + | + | - | + |
| 14 | - | + | + | - | + |
| 15 | + | + | + | + | (+) |

## Patentansprüche

1. Streptolysin O Peptidantigen, welches im CD-Spektrum eine negative Bande zwischen 190 und 200 nm zeigt und mindestens eine der Aminosäuresequenzen SEQ ID NO: 1 - 5 oder Teilsequenzen davon mit mindestens 4 Aminosäuren Länge enthält.

2. Streptolysin O Peptidantigen nach Anspruch 1 mit der Aminosäuresequenz SEQ ID NO: 1, 2, 3, 4 und/oder 5.

3. Streptolysin O Peptidantigen nach Anspruch 2 mit Teilsequenzen von maximal 7 Aminosäuren Länge.

4. Streptolysin O Peptidantigen nach Anspruch 2 mit den Teilsequenzen PESSELTTE aus SEQ ID NO: 1, TDDMLNSND aus SEQ ID NO: 2, PLESAEK aus SEQ ID NO: 4 und YDDKGKEVI aus SEQ ID NO: 5.

5. Kombination von Streptolysin O Peptidantigenen aus SEQ ID NO: 2 und/oder 5 mit SEQ ID NO: 1, 3 und/oder 4 oder Teilsequenzen davon mit mindestens 4 Aminosäuren Länge.

6. Verfahren zur Herstellung von Peptidantigenen nach den Ansprüchen 1 bis 4, dadurch gekennzeichnet, daß die das C-terminale Ende bildende Aminosäure an einen Träger gebunden wird, vom C-terminalen Ende das Peptidantigen schrittweise aufgebaut und anschließend vom Träger abgespalten wird.

7. Verfahren zur Bestimmung von Anti-SLO-Antikörpern, dadurch gekennzeichnet, daß die Probe mit den Peptidantigenen oder Teilsequenzen davon mit mindestens 4 Aminosäuren Länge der SEQ ID NO: 2 und/oder 5 oder mit der Kombination der Peptidantigene SEQ ID NO: 1, 3 und 4 inkubiert und unter Bedingungen, die die Bildung eines Antikörper-Antigenkomplexes ermöglichen, die Menge der an das Peptidantigen gebundenen SLO-Antikörper bestimmt wird.

8. Verfahren nach Anspruch 7, dadurch gekennzeichnet, daß mindestens ein Peptidantigen mit 4 - 7 Aminosäuren Länge, welches eine Teilsequenz von SEQ ID NO: 1 - 5 darstellt, verwendet wird.

9. Verfahren nach Anspruch 7, dadurch gekennzeichnet, daß aus SEQ ID NO: 1 PESSELTTE, aus SEQ ID NO: 2 TDDMLNSND, aus SEQ ID NO: 4 PLESAEK und/oder aus SEQ ID NO: 5 YDDKGKEVI als Teilsequenzen verwendet werden.

10. Verfahren zur Bestimmung von Anti-SLO-Antikörpern nach einem der Ansprüche 7 - 9, dadurch bekennzeichnet, daß es nach dem FPIA, CEDIA, EMIT, LPIA oder ELISA-Prinzip durchgeführt wird.

11. Verfahren zur Herstellung von Antikörpern gegen SLO-Antigene, dadurch gekennzeichnet, daß ein Säugetier mit einem gegebenenfalls trägergebundenen Peptidantigen nach Anspruch 1 - 4 immunisiert wird, polyklonale Antikörper gewonnen werden oder Zellen dieser Tiere, die Antikörper produzieren, zu Zellinien immortalisiert werden und aus diesen Zellinien monoklonale Antikörper gewonnen werden.

12. Verfahren nach Anspruch 11, dadurch gekennzeichnet, daß als Peptidantigene Peptidantigene nach Anspruch 1 - 4 verwendet werden.

13. Verfahren zur Bestimmung von SLO, dadurch gekennzeichnet, daß die Probe mit einem Antikörper erhältlich nach Anspruch 11 oder 12 unter Bedingungen, die eine Antigen-Antikörperkomplexbildung erlauben, inkubiert wird, und die Menge des gebildeten Antikörper-Antigenkomplexes bestimmt wird.

14. Verfahren zur Bestimmung von SLO nach Anspruch 13, dadurch gekennzeichnet, daß es nach dem FPIA, CEDIA, EMIT, ELISA oder Agglutinations-Prinzip durchgeführt wird.

15. Verwendung der Peptidantigene nach einem der Ansprüche 1 - 4 als Standard in einem Immunoassay zum Nachweis von SLO.

16. Verwendung von Antikörpern gegen SLO-Antigene erhältlich nach einem der Ansprüche 11 oder 12 als Standard in einem Immunoassay zum Nachweis von Anti-SLO-Antikörpern.

17. Verfahren zur Herstellung von Impfstoffen gegen Streptokokken-Infektionen unter Verwendung der Peptidantigene nach Anspruch 1 - 4 als Immunogene.
